# EUROPEAN PATENT SPECIFICATION

(11) **EP 4 314 160 B1**
(45) Date of publication and mention of the grant of the patent: **09.12.2026**
(21) Application number: 22733046.1
(22) Date of filing: 09.06.2022
(51) Int. Cl.: C08L 89/06, C07K 14/78, C12N 5/00

(54) **CROSSLINKABLE FUNCTIONALISED-GELATIN HAVING LOW PYROGENIC ACTIVITY**
VERNETZBARE FUNKTIONALISIERTE GELATINE MIT GERINGER PYROGENER WIRKUNG
GÉLATINE FONCTIONNALISÉE RÉTICULABLE À FAIBLE ACTIVITÉ PYROGÉNIQUE

(30) Priority: 10.06.2021 BE 202105461; 09.07.2021 BE 202105534
(43) Date of publication of application: 07.02.2024
(73) Proprietor: Rousselot BV, 9000 Gent (BE)
(72) Inventor: VERGAUWEN, Bjorn, 9000 GENT (BE)
(74) Representative: EP&C
(86) International application number: PCT/EP2022/065716
(87) International publication number: WO 2022/258763

(56) References cited:
- WO-A1-2016/085345
- WO-A1-2020/050779
- ROCH TORALF ET AL: "Reducing the Endotoxin Burden of Desaminotyrosine- and Desaminotyrosyl Tyrosine-Functionalized Gelatin", 1 January 2011 (2011-01-01), pages 182 - 189, XP093196142, Retrieved from the Internet <URL:https://www.researchgate.net/publication/264687986_Reducing_the_Endotoxin_Burden_of_Desaminotyrosine-_and_Desaminotyrosyl_Tyrosine-Functionalized_Gelatin>
- WANG L S ET AL: "Injectable biodegradable hydrogels with tunable mechanical properties for the stimulation of neurogenesic differentiation of human mesenchymal stem cells in 3D culture", BIOMATERIALS, ELSEVIER, AMSTERDAM, NL, vol. 31, no. 6, 1 February 2010 (2010-02-01), pages 1148 - 1157, XP026814142, ISSN: 0142-9612, [retrieved on 20091104]

## Description

### Field of the invention

The application generally relates to chemically functionalized gelatin, in particular gelatin functionalised with a cross-linkable, substituted carboxylic acid. More particularly, the invention relates to cross-linkable, substituted carboxylic acid-functionalised -gelatin with low pyrogenic activity, in particular low lipopolysaccharide and low residual free carboxylic acid R- (CH2)n-COOH content, hydrogels comprising cross-linkable, substituted carboxylic acid-functionalised -gelatin, a method for preparing cross-linkable, substituted carboxylic acid-functionalised -gelatin and uses thereof

### Background of the invention

Reconstruction of functional biological tissue, biological implants, and cell-based multiorgan models for clinical, diagnostic or pharmaceutical research receive high attention. Hydrogels have emerged as leading candidates for various tissue engineering applications due to their similarity with the native extracellular matrix.

Gelatin hydrogels are particularly attractive because of their biocompatibility and biodegradability. Gelatin is produced by the partial hydrolysis of collagen, the most abundant protein in the body and the most prevalent molecule of the extracellular matrix. The abundant presence of the cell-recognition sequence Arginine-Glycine-Aspartic acid (RGD) facilitates attachment of cells to gelatin promoting their spreading and proliferation. These cell-matrix interactions are crucial for the organization of complex tissue. Thanks to the long history of gelatin as a trusted excipient within the pharmaceutical industry, it meets the highest standards of safety and regulatory compliance. Furthermore, the presence of free amino groups, hydroxyl groups and carboxylate groups enable chemical modification to achieve desired properties for specific applications.

Gelatin hydrogels are fabricated by crosslinking of gelatin polymers, either without prior modification or after functionalization of their side groups. The addition of functional groups to the gelatin backbone is a crosslinking strategy with a high degree of control over hydrogel design and properties. The most extensively used and studied modification for crosslinking gelatin is methacryloylation. MA-modified gelatin is generally referred to as gelMA. An alternative to the well-established methacryloyl-gelatin is acryloyl-gelatin (Billiet et al. 2013 "Quantitative Contrasts in the Photopolymerization of Acrylamide and Methacrylamide-Functionalized Gelatin Hydrogel Building Blocks" Macromolecular Bioscience 13:1531-45).

Other functionalised gelatines are for instance tyramine-functionalised gelatins (Wang et al., 2010, Biomaterials. 31(6):1148-57). Wang et al. discloses the functionalisation of gelatin through modification with a combination of 3-(4-hydroxyphenyl)-propionic acid (HPA), an excess of N-hydroxysuccinimide (NHS) and 1-ethyl-3-(3-dimethylaminopropyl)-carbodiimide hydrochloride (EDC). WO2006/010066 discloses modification of gelatin using a carbodiimide mediated coupling of tyramine to gelatin. Other related publications are WO2020/050779, disclosing hydrogels with tuneable properties based on tyramine or hydroxyphenyl propionic acid based crosslinking agents. The compound 3-(4-hydroxyphenyl)propanoic acid (HOC₆H₄(CH₂)₂CO₂H is sometimes abbreviated as HPA an also known as phloretic acid or desaminotyrosine (sometimes abbreviated as DAT). These indications may be used interchangeably.

Elvin et al. 2010, Biomaterials. 31(6):8323-8331 describes the generation of a photopolymerisable gelatin-based material based on increasing the linkable moiety parahydroxylphenyl (tyrosine-like) content of the gelatin by coupling of free amines with 3-(4-hydroxyphenyl)propanoic acid. The DAT content was increased by reaction of the gelatin with a Bolton-Hunter reagent (*N*-succinimidyl-3-[4-hydroxyphenyl]propionate). This is an example of a carboxylic acid-functionalised -gelatin. Also Roch Toralf ET AL: "Reducing the Endotoxin Burden of Desaminotyrosine- and Desaminotyrosyl Tyrosine-Functionalized Gelatin", (2011-01-01), pages 182-189 discloses the preparation of a carboxylic acid modified gelatin.

Depending on the degree of functionalisation and the polymer concentration, the physical properties (crosslinking density, swelling and stiffness) of the gelatin-based hydrogels can be tailored, which makes this material a versatile platform for various tissue engineering applications. The crosslinking can be initiated in a variety of ways. One of these methods is by radicals which are generated by UV or visible light depending on the photoinitiator used. Alternatively via thiol-ene photo click chemistry, thiol-Michael addition, inverse electron demand Diels-Alder reaction, Diels-Alder Click reaction, disulphide bridge formation, Schiff/'s base, pi-pi cycloaddition, photo oxidation and/or enzymatic crosslinking.

One of the major drawbacks of gelatin-based hydrogels for (bio)medical applications is the presence of endotoxins (also referred to herein as lipopolysaccharides) in traditionally manufactured gelatin. Endotoxins are large, highly immunogenic molecules and the major component of the outer membrane of gram-negative bacteria. They are highly heat resistant, making them difficult to inactivate. When exposed to the immune system, endotoxins initiate an immune response, which can lead to tissue inflammation, increased sensitivity to other allergens, and the risk for fatal shock. Most research is currently conducted on crosslinked functionalised gelatines that is based on gelatin with high endotoxin levels.

Traditionally manufactured gelatin can also be contaminated by microbial components other than endotoxins, some of which can, like endotoxins, cause an adverse immune response in humans. Non-endotoxin pyrogens include, for example, substances such as lipoteichoic acid (LTA) originating from Gram-positive bacteria, and other compounds originating from fungi, yeast, viruses, bacteria, and parasites (Hasiwa et al. (2013) "Evidence for the detection of nonendotoxin pyrogens by the whole blood monocyte activation test. ALTEX 30:169-208). These non-endotoxin pyrogens, and preferably pyrogens or Pathogen-Associated Molecular Patterns (PAMPs) in general, should also be kept to a minimum for (bio)medical applications of gelatin-based hydrogels to prevent unwanted side effects upon activation of innate immunereceptors.

In addition, during the functionalization and crosslinking of gelatin to gelatin hydrogel, a number of reagents are used. Residues of reagents and reaction products can be present in the functionalised gelatin. For instance, upon reacting gelatin a N-succinimidyl-3-[4-hydroxyphenyl]propionate such as described by Elvin et al.(see above), hydrolysis products such as 3-[4-hydroxy]propionic acid (HPA, phloretic acid) and unreacted reagents such as N-succinimidyl-3-[4-hydroxyphenyl]propionate can be present in the functionalised gelatin composition. These remnants are considered undesirable. Other problems to solve with chemically modified gelatin is that the chemical process may lead to deterioration or hydrolysis of the gelatin (lowering of the Mw) or to undesired crosslinking (increased molecular weight). It is hence desirable that the chemical modification results in a gelatin that has about the same Mw prior and after functionalisation.

Conventional procedures rely on purifying the reaction mixture by dialysis against distilled water. Typically, dialysis is performed for one week. This time-consuming step is therefore not efficient for large-scale production of functionalised gelatins. Furthermore, during this week of dialysis, the risk for microbial contamination and gelatin degradation increases. For (bio)medical applications of gelatin-based hydrogels, there is a need for biocompatible functionalised gelatins (e.g. non-toxic to biological tissue and nonimmunogenic), which allows to tailor hydrogels with desired mechanical properties (for example, strength and elasticity). There is also a need for manufacturing such gelatins by an efficient process allowing production at industrial scale.

### Summary of the invention

The present invention solves one or more of the above described problems of the prior art. In particular, carboxylic acid modified -gelatin (modified gelatin comprising primary amines functionalised with a substituted carboxylic acid) is provided that has a low pyrogen content, in particular a low endotoxin content, and low contamination of reagents, side products and hydrolysis products. The carboxylic acid-functionalised -gelatin has improved biocompatibility due to the low pyrogen content, in particular the low endotoxin content. The carboxylic acid-functionalised -gelatin of the present invention is particularly useful for crosslinking due to the low contamination with reactants, which are known to interfere with the crosslinking process. Also advantageously, the carboxylic acid-functionalised -gelatin can be prepared by a simple manufacturing process that does not require a lengthy dialysis step. Disclosed herein are one or any combination of one or more of the below numbered aspects and embodiments (i) to (xv) wherein:
(i) Gelatin functionalised via an amide bond with a carboxylic acid moiety R-(CH₂)ₙ-COOH wherein n is an integer form 1 to 10 and further having a lipopolysaccharide content of less than 100 EU/g. 100 EU/g, preferably less than 50 EU/g, more preferably less than 20 EU/g, even more preferably less than 10 EU/g, even more preferably less than 5 EU/g, even more preferably less than 2 EU/g, most preferably less than 1 EU/g.
(ii) Gelatin according to (i), comprising less than 100 ppm of reactants and reagents, preferably free, substituted, carboxylic acid.
(iii) Gelatin according to (i) or (ii), having a low content of pyrogens from Gram+ bacteria, pyrogens from flagellated bacteria, single-stranded viral RNA and bacterial DNA rich in unmethylated CpG motifs, preferably a low content of pyrogens from Gram+ bacteria and from flagellated bacteria, more preferably a low content of pyrogens from Gram+bacteria.
(iv) Gelatin according to any one of (i) to (iii), wherein said gelatin is type A gelatin.
(v) Gelatin according to any one of (i) to (iv), having a degree of carboxylic acid substitution of between 5% and 100%, preferably between 25% and 100%, more preferably between 50 and 100%, even more preferably between 80% and 100%.
(vi) Gelatin according to any one of (i) to (v), wherein said gelatin is further modified with further modified with an (meth)acryloyl group or moiety, an acetyl group or moiety, a phenol group or moiety, a thiol group or moiety, a norbornene group or moiety, a tetrazine group or moiety, an azide group or moiety, a furan group or moiety, an allyl group or moiety, a maleimide group or moiety or any combination thereof
(vii) Hydrogel comprising a gelatin modified with a carboxylic acid according to any one of (i) to (vi), and a cross-linking agent.
(viii) A film comprising a hydrogel according to (vii) or a gelatin according to any one of (i) to (vi),
(ix) Method for providing a functionalised gelatin according to any one of (i) to (vi),, the method comprising the steps of
   a. modifying gelatin by reacting gelatin with a reagent containing an N-hydroxysuccinimide ester of the carboxylic acid moiety R-(CH₂)ₙ-COOH wherein n is an integer from 1 to 10 (preferably R-(CH₂)ₙ-COO-NHS) in a reaction medium;
   b. lowering the pH of the reaction medium to a value between 2.0 and 4.0, preferably between 2.5 and 3.5, more preferably between 3.0 and 3.5;
   c. adding 0.01- 1.5 w/w% of a micelle-forming surfactant to the acidic reaction medium;
   d. contacting the micelle-containing medium with an adsorbent, preferably a solid adsorbent, preferably a solid adsorbent, preferably activated carbon;
   e. separating the adsorbent from the medium;
   f. recovering the medium comprising the functionalised gelatin.
(x) The method according to (ix), wherein said micelle-forming surfactant comprises a nonionic surfactant, preferably wherein the surfactant is Triton X-100 or Triton X-102, or mixtures thereof.
(xi) The method according to (ix) or (x), wherein said solid adsorbent is a hydrophobic adsorbent, preferably activated carbon.
(xii) The method according to any one of (ix) to (xi), further comprising a step of drying the medium comprising the carboxylic acid modified gelatin.
(xiii) Gelatin according to any one of (i) to (vi) or a hydrogel according to (vii) for use in medicine.
(xiv) In vitro or ex vivo use of gelatin according to any one of (i) to (vi) or a hydrogel according to (vii) for manufacturing a biological construct such as a tissue or an organ, or a part thereof, a coating, a scaffold, or a controlled release dosage form.
(xv) Use of gelatin according to any one of (i) to (vi) or a hydrogel according to (vii) as a bio-ink or bio-resin.

### Brief description of the Figures

Fig 1 Graphic representation of the purification of 3-(4-hydroxyphenyl)-propionic acid modified gelatin using dialysis resulting in a reduction of 98% after dialysis at T=5.
Fig 2 Graphic representation of the purification of 3-(4-hydroxyphenyl)-propionic acid modified gelatin using micelle formation, absorption and filtration.
Fig 3 HPLC signal detected for the AC filtration samples. PA is not detected.
Fig 4 MW distribution for the starting gelatin (1) and the results of a modification via a route using an in situ mixture of NHS, HPA and EDC followed by purification using one step dialysis (2) and two-step dialysis (3).
Fig 4B MW distribution for the same starting gelatin (1) and the results of a modification via a route using an NHS-HPA in essential absence of EDC, followed by purification using a two-step purification based on micelle formation.

### Detailed description

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or openended and do not exclude additional, non-recited members, elements or method steps. Where reference is made to embodiments as comprising certain elements or steps, this encompasses also embodiments which consist essentially of the recited elements or steps. The recitation of numerical ranges by endpoints includes all numbers and fractions subsumed within the respective ranges, as well as the recited endpoints. The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The present application generally relates to functionalized gelatin for cross-linking gelatin to prepare e.g. gelatin-based hydrogels and films. More particularly, the application relates to carboxylic acid-functionalised -gelatin.

Gelatin is a mixture of water-soluble proteins derived from collagen. Gelatin is obtained e.g. by partial hydrolysis of collagen, obtained by aqueous extraction of skin, tendons, ligaments, bones etc., from e.g. bovine, porcine, poultry or fish, in acid or alkali conditions, or by enzymatic hydrolysis, as known in the art. Gelatin obtained by acid treatment is called "type A gelatin", whereas "type B gelatin" is derived from alkali based process. Due to a more extensive deamination of asparagine and glutamine in type B gelatin, the isoelectric points (IEP) of type A gelatin and type B gelatin are at pH 7.0-9.0 and pH 4.9-5.1, respectively, which enables them to be positively and negatively charged at neutral physiological pH. In preferred embodiments, the invention relates to type A gelatin.

Gelatin does not constitute a uniform protein molecule, but comprises a variable amount of protein molecules of variable length. Preferably, the gelatin used herein has an average molecular weight within the range of 1500 Da to 300 kDa, preferably between 2000 Da and 300 kDa, between 4000 Da and 300 kDa, between 5000 Da and 300 kDa, between 10 kDa and 300 kDa, or between 20 kDa and 300 kDa, more preferably between 50 kDa and 300 kDa, most preferably between 100 kDa and 300 kDa, such as between 100 kDa and 275 kDa or between 100 kDa and 250 kDa. The molecular weight distribution of gelatin is usually measured by size exclusion high performance liquid chromatography (HPLC) techniques, and eluted fractions are detected by UV adsorption and the measured data are evaluated by suitable software, all techniques, known in the art, see e.g. Olijve et.al. (2000. Journal of Colloid and Interface Science 243: 476-482).

As used herein, the term "gelatin" also encompasses "gelatin derivatives", including chemically modified gelatin. The expression "gelatin modified with a chemical group or moiety (e.g. a carboxylic group or moiety, a tyramine group or moiety, a methacryloyl group or moiety, an acryloyl group or moiety, an acetyl group or moiety, a phenol group or moiety, etc.)" as used herein denotes gelatin comprising said chemical group or moiety (e.g. a carboxylic acid group, a tyramine group or moiety, a methacryloyl group or an acryloyl group, an acetyl group or moiety, a phenol group or moiety, etc.) e.g. attached to at least one amine group, at least one hydroxyl group, at least one carboxyl group and/or at least one phenol group of the gelatin.

As used herein "gelatin modified with a carboxylic acid group", or "gelatin modified with a cross- linkable, substituted carboxylic acid group", also referred to herein as "carboxylic acid -modified gelatin", "carboxylic acid -substituted gelatin" or " carboxylic acid -gelatin" or 'gelatin carboxylic acid" is defined as gelatin having free amines that have been substituted with at least one carboxylic acid group, preferably via an amide bond. Gelatin comprises amino acids, some of which have side chains that contain terminal amines (e.g., lysine, arginine, asparagine, glutamine) or hydroxyls (e.g., serine, threonine, aspartic acid, glutamic acid, tyrosine, hydroxyproline). Furthermore, gelatin also contains N-terminal amines. All of these terminal amines can be substituted with carboxylic acid groups to produce functionalized -gelatin comprising carboxylic acid groups. There is a preference for the substitution of N-terminal amines wit carboxylic acids to form an amide bond.

The carboxylic acid used in the functionalisation of the gelatin of the present invention is a carboxylic acid having the formula R-(CH₂)ₙ-COOH wherein n is an integer form 1 to 10.

In embodiments, in the carboxylic acid used in the functionalisation of the gelatin, R is selected from the group consisting of 4-phenol, norbornenyl or SH. In embodiments, wherein n is from 1 to 5, preferably 2 or 3. In preferred embodiments, in the carboxylic acid used in the functionalisation of the gelatin, the carboxylic acid moiety is one or more of 3-(4-hydroxyphenyl)-propionic acid, 3-(SH)-propionic acid, and 2-(5-norbornenyl)-acetic acid, preferably 3-(4-hydroxyphenyl)-propionic acid.

The "degree of functionalization (DoF)" of gelatin generally refers to the percentage of functionalized primary amine groups over total primary amine groups
. As used herein, the "degree of carboxylic acid substitution" refers to the percentage of free amine groups in the gelatin that have been substituted with a carboxylic acid group.
The degree of functionalization such as the degree of carboxylic acid substitution of gelatin can be determined by methods known per se.

For example, the Fe(III)-acetohydroxamic acid method can be used for determining substitution at hydroxyl groups. The Habeeb method (trinitrobenzenesulfonic acid (TNBS)-based spectrophotometric determination of (meth)acrylamide; Habeeb 1996. Anal. Biochem. 14:328-336), 1H-NMR and the fluoraldehyde assay (also known as o-Phthaldialdehyde (OPA)-based fluorometric determination of free amines can be used for determining carboxylic acid substitution at amine groups. One can also use a combination of the aforementioned methods, such as a combination of a fluoraldehyde assay for quantifying amine group conversion and a Fe(III)-acetohydroxamic acid method for quantification of hydroxyl groups groups,. In embodiments, the fluoraldehyde assay is used for determining the degree of carboxylic acid substitution by determination of the free amines.

In the carboxylic acid-functionalised -gelatin disclosed herein, carboxylic acid - functionalisation is preferably at the free amine groups. In embodiments, the carboxylic acid-functionalised -gelatin has a degree of carboxylic acid substitution of between 20% and 100%, preferably between 50% and 100%, more preferably between 80% and 100% such as between 85% and 100%, between 90% and 100 or between 95% and 100%. In embodiments, the carboxylic acid-functionalised -gelatin has a degree of carboxylic acid substitution of less than 20%, preferably less than 10%, 9%, 8%, 7% or 6%, more preferably less than 5%, 4%, 3%, 2% or 1%.

The carboxylic acid-functionalised -gelatin disclosed herein may be further modified and/or functionalized. In embodiments, the carboxylic acid -gelatin is further modified with an a (meth)acryloyl group or moiety, an acetyl group or moiety, a phenol group or moiety, a thiol group or moiety, a norbornene group or moiety, a tetrazine group or moiety, an azide group or moiety, a furan group or moiety, an allyl group or moiety, a maleimide group or moiety or any combination thereof, preferably an acetyl group. Double chemically functionalized gelatin may be produced as described in Hoch et al. (2012. Chemical tailoring of gelatin to adjust its chemical and physical properties for functional bioprinting. J. Mater. Chem. B 1:5675).

The carboxylic acid -gelatin disclosed herein have low pyrogenic activity. Pyrogenic activity can be measured using a Monocyte Activation Test (MAT) assay as known in the art. The MAT assay allows to quantify both endotoxin and non-endotoxin pyrogen levels, but does not discriminate between the type of PAMP contamination. A cell-based pyrogen detection assay (PAMP assay) was developed by Fraunhofer Institute for Interfacial Engineering and Biotechnology IGB for detection and quantification different PAMPs, also non-endotoxins. This cell-based test system detects PAMPs using human Toll-like receptors (TLRs) (Burger-Kentischer et al. (2010) A new cell-based innate immune receptor assay for the examination of receptor activity, ligand specificity, signaling pathways and the detection of pyrogens. J Immunol Methods, 358: 93-103). More particularly, cells, e.g. NIH3T3 cells, express different TLR combinations allowing for the detection of different PAMPs. For example, cell lines with the receptor combination TLR1/2, TLR2/6, TLR4/CD14, TLR5, TLR7 and TLR9 allow for the detection of pyrogens from Gram+ bacteria (TLR1/2, TLR2/6), pyrogens from flagellated bacteria (TLR5), single-stranded viral RNA (TLR7), the cell line TLR4/CD14 allows for the detection of endotoxin from Gram- bacteria and the TLR9 cell line detects bacterial DNA rich in unmethylated CpG motifs. In embodiments, the HPA-gelatins disclosed herein have a low content of pyrogens from Gram+ bacteria, pyrogens from flagellated bacteria, single-stranded viral RNA, endotoxin from Gram- bacteria and bacterial DNA rich in unmethylated CpG motifs.

In particular embodiments, the carboxylic acid -gelatin has a low content of pyrogens from Gram+ bacteria and from flagellated bacteria, more particularly a low content of pyrogens from Gram+ bacteria In particular embodiments, the carboxylic acid -gelatin disclosed herein are characterized by a low endotoxin or lipopolysaccharide (LPS) content, in particular an LPS content of less than 100 EU/g, more preferably less than 50 EU/g, even more preferably less than 20 EU/g, even more preferably less than 10 EU/g, even more preferably less than 5 EU/g, even more preferably 5 less than 2 EU/g, most preferably less than 1 EU/g. In further particular embodiments, carboxylic acid -gelatin disclosed herein is (derived from) a type A gelatin and is characterized by an LPS content of less than 100 EU/g, more preferably less than 50 EU/g, even more preferably less than 20 EU/g, even more preferably less than 10 EU/g, even more preferably less than 5 EU/g, even more preferably less than 2 EU/g, most preferably less than 1 EU/g. In other further particular embodiments, the carboxylic acid -gelatin disclosed herein is (derived from) a type B gelatin and is characterized by an LPS content of less than less than 20 EU/g, preferably less than 10 EU/g, more preferably less than 5 EU/g, even more preferably less than 2 EU/g, most preferably less than 1 EU/g. The term EU is known in the art and reflects 'endotoxin units'. One EU is approximately equivalent to 100 pg of E. coli lipopolysaccharide, the amount present in about 104-105 bacteria. Herein, the term EU/g reflects the EU count per dry weight of carboxylic acid-functionalised -gelatin. The Limulus assay (LAL) is a well-known bioassay in the art to measure up to subpicogram quantities of LPS. Limulus amebocyte lysate (LAL) is an aqueous extract of blood cells (amoebocytes) from the horseshoe crab, Limulus polyphemus. LAL reacts with bacterial endotoxin or lipopolysaccharide. This reaction is the basis of the LAL test, which is then used for the detection and quantification of bacterial endotoxins. For instance, a suitable LAL method to quantify the LPS levels is the chromogenic Endosafe method, e.g. from Charles River USA. Other accepted and recommended methods are the EndoZyme recombinant factor C method from Hyglos GmbH (Germany). Both said methods result in similar or identical measurement values and can therefore be used interchangeably.

Also advantageously, the carboxylic acid -gelatin disclosed herein is substantially free of reagents and reaction products. The carboxylic acid -gelatin disclosed herein comprises a low level of free carboxylic acid, i.e. carboxylic acid that is not bound to gelatin, of less than 150 ppm carboxylic acid or less than 100 ppm carboxylic acid or less than 50 ppm carboxylic acid, preferably less than 30 ppm carboxylic acid, more preferably less than 25 ppm carboxylic acid, even more preferably less than 20 ppm carboxylic acid. In embodiments, the carboxylic acid-gelatin disclosed herein comprises less than 150 ppm carboxylic acid such as less than 140 ppm carboxylic acid, less than 130 ppm carboxylic acid, less than 120 ppm carboxylic acid or less than 110 ppm carboxylic acid, preferably less than 100 ppm carboxylic acid such as less than 90 ppm carboxylic acid, less than 80 ppm carboxylic acid, less than 70 ppm or less than 60 ppm, wherein, preferably, the carboxylic acid content is determined on a sample of the carboxylic acid-gelatin dissolved in 50 mM phosphate buffer, pH 9.5. Carboxylic acid can be measured as detailed in the Examples, in particular by dissolving a sample of the carboxylic acid -gelatin in water or in 50 mM phosphate buffer, pH 9.5, ultrafiltration of the dissolved sample (e.g. using 10-kDa Amicon Ultra Centrifugal Filters) and HPLC analysis of the filtrate. Residues of carboxylic acid in the carboxylic acid-functionalised -gelatin may interfere with the crosslinking of carboxylic acid-functionalised -gelatin molecules. Due to its low carboxylic acid content, the carboxylic acid-functionalised -gelatin disclosed herein is thus particularly suitable for crosslinking to form a hydrogel or a film. Accordingly, a further aspect relates to a hydrogel comprising crosslinked carboxylic acid -gelatin or crosslinked carboxylic acid -gelatin as disclosed herein. Further disclosed herein are products derived from said hydrogels, such as films, bioadhesives, etc. The term "hydrogel" as used herein refers to a network of hydrophilic polymer chains, such as crosslinked carboxylic acid -gelatin, forming a gel. The term "gel" denotes a substantially dilute crosslinked system which exhibits no flow when in the steady-state.

Processes for crosslinking carboxylic acid -gelatin are well-known in the art. Typically, upon exposure to light in the presence of a photoinitiator, the carboxylic acid groups on one gelatin molecule can react with the carboxylic acid groups on another gelatin molecule to crosslink the carboxylic acid - gelatin.

The term "photoinitiator" as used herein refers to any chemical compound, or a mixture of compounds, that decomposes into free radicals when exposed to light, e.g. ultraviolet light (UV) or visible light (VIS). Non-limiting examples of ultraviolet photoinitiators include 1-[4-(2-hydroxyethoxy)-phenyl]-2-hydroxy-2-methyl-1-propane-1-one (also known under the trade name Irgacure^{®} 2959) and lithium phenyl-2,4,6-tri-methylbenzoylphosphinate (LAP). Visible light photoinitiators produce free radicals when exposed to visible light. Exemplary ranges of visible light useful for exciting a visible light photoinitiator include green, blue, indigo, and violet. Preferably, the visible light has a wavelength in the range of 450-550 nm. Non-limiting examples of visible light photoinitiators include, Eosin Y, riboflavin/triethanolamine, vinyl caprolactam, dl-2,3-diketo- 1,7,7-trimethylnorcamphane (CQ), 1 -phenyl- 1 ,2-propadione (PPD), 2,4,6-trimethylbenzoyl- diphenylphosphine oxide (TPO), bis(2,6-dichlorobenzoyl)-(4-propylphenyl)phosphine oxide (Ir819), 4,4'-bis(dimethylamino)benzophenone, 4,4'- bis(diethylamino)benzophenone, 2-chlorothioxanthen-9-one, 4-(dimethylamino)benzophenone, phenanthrenequinone, ferrocene, diphenyl 1(2,4,6 trimethylbenzoyl)phosphine oxide / 2-hydroxy-2-methylpropiophenone (50/50 blend), dibenzosuberenone, (benzene) tricarbonylchromium, resazurin, resorufin, 5 benzoyltrimethylgermane (Ivocerin^{®}), derivatives thereof, and any combination thereof.

The light irradiation time may be any suitable time for enabling crosslinking of the polymer. For example, the irradiation time may range from 10 seconds to 20 minutes, preferably from 1 minute to 20 minutes, more preferably from 2-15 minutes (for example 2, 3, 4, 5, 6, 7, 8, 9, 10, 11, 12, 13, 14 or 15 minutes).

The mechanical properties of a gelatin-based hydrogel can be tuned for various applications e.g.by the molecular weight of the gelatin used, by changing the degree of HPA substitution, carboxylic acid -gelatin concentration, amount of photoinitiators, and light exposure time.

As used herein, the concentration of carboxylic acid -substituted gelatin is defined as the weight of carboxylic acid -substituted gelatin divided by the volume of solvent (w/v), expressed as a percentage. The solvent may be a pharmaceutically acceptable carrier. In embodiments of the hydrogel, the carboxylic acid -substituted gelatin is present at a concentration between 5% and 25% (w/v), between 17% and 25% (w/v), between 17% and 23% (w/v), or about 20% (w/v). In some embodiments, the carboxylic acid -substituted gelatin is present at a concentration between 5% and 15% (w/v), between 8% and 12% (w/v), or about 10% (w/v). In some embodiments, the carboxylic acid -substituted gelatin is present at a concentration between 10% and 40% (w/v), 15% and 35% (w/v), 20% and 30% (w/v), or about 5%, 10%, 15%, 20%, or 25% (w/v).

The chemically modified gelatin, in particular the carboxylic acid -gelatin, and the hydrogel according to the invention may be used for a variety of applications including, but not limited to, the manufacture or repair of tissue (e.g. cartilage, soft tissue) in a human or nonhuman animal, and the use as a bio-ink or bio-resin for the 3-dimensional biofabrication or 3- dimensional bioprinting of a biological construct. The biological construct may be any animal tissue or organ, or part thereof, that is able to be manufactured using a biofabrication or bioprinting technique, e.g. a scaffold containing cells which may be porous or non-porous.

The term "bio-ink" refers to a hydrogel that can be 3D-printed, 3D-plotted or fabricated into a particular shape or construct, and which is cytocompatible. The hydrogel may or may not incorporate living cells and/or growth factors, etc. he term "bio-resin" denotes a hydrogel that can be 3D-printed or fabricated into a particular shape or construct using laser or light projection-based light stereolithography, or similar lithographic techniques, and which is cytocompatible. The hydrogel may or may not incorporate living cells, drugs and/or growth factors, etc.

carboxylic acid A related aspect is directed to in vitro or ex vivo use of a carboxylic acid-gelatin disclosed herein or a hydrogel comprising the carboxylic acid-gelatin disclosed herein for manufacturing a tissue or an organ, or a part thereof. In embodiments, the tissue or organ is selected from bone tissue, cartilage and vascular tissue. Also disclosed herein is a tissue- engineered tissue or organ, or part thereof, comprising a carboxylic acid-gelatin disclosed herein or a hydrogel comprising the carboxylic acid-gelatin disclosed herein.

Another aspect is directed to in vitro or ex vivo use of a carboxylic acid-gelatin disclosed herein or a hydrogel comprising the carboxylic acid-gelatin disclosed herein for the manufacture of a controlled release dosage form or a biological construct. The biological construct can be, for example, a coating on a (solid) support suitable for adhesion and proliferation of cells, or a scaffold suitable for containing cells, or drugs and/or vectors.

Yet a further aspect relates to uses of a carboxylic acid-gelatin disclosed herein or a hydrogel comprising the carboxylic acid-gelatin disclosed herein as a bio-ink or bio-resin.

In further embodiments, the bio-ink or bio-resin is used for 3-dimensional biofabrication or 3- dimensional bioprinting of a biological construct. The biological construct may be an animal tissue or organ, or part thereof. The biological construct can also be a scaffold suitable for containing cells, or a scaffold suitable for containing e.g. drugs and/or vectors (e.g. for drug delivery/gene therapy applications). The biological construct can also be a bioresorbable screw or other biomaterial (e.g. bioadhesive). In embodiments, the biological construct is a scaffold suitable for containing cells. In embodiments, the biological construct is a coating. Also disclosed herein, is a biological construct comprising the carboxylic acid-gelatin disclosed herein or a hydrogel comprising the carboxylic acid-gelatin disclosed herein. Yet a further aspect relates to a method for preparing a carboxylic acid-gelatin as disclosed herein. The present inventors have surprisingly found that purification of an aqueous reaction medium of carboxylic acid-gelatin according to a modified version of the method described in WO 2016/085345, not only removes lipopolysaccharides from the medium, but also other pyrogens as well as the free carboxylic acid and other reactants that are formed during or that remain after the carboxylic acid functionalisation of the gelatin reaction. As such, no dialysis of the reaction medium is required, resulting in a faster process that provides carboxylic acid-gelatin that has a low LPS content, a low pyrogen content and a low carboxylic acid content as specified elsewhere herein.

Accordingly, in a further aspect, the invention relates to a method of preparing a carboxylic acid-gelatin as disclosed herein, said method comprising the following steps:
a. modifying gelatin by reacting gelatin with a reagent containing an N-hydroxysuccinimide ester of the carboxylic acid moiety R-(CH₂)ₙ-COOH wherein n is an integer from 1 to 10 (R-(CH₂)ₙ-COO-NHS) in a reaction medium;
b. lowering the pH of the reaction medium to a value between 2.0 and 4.0, preferably between 2.5 and 3.5, more preferably between 3.0 and 3.5;
c. adding 0.01- 1.5 w/w% of a micelle-forming surfactant to the acidic reaction medium;
d. contacting the micelle-containing medium with an adsorbent, preferably activated carbon;
e. separating the adsorbent from the medium;
f. recovering the medium comprising the functionalised gelatin.
g. Optionally, drying the medium comprising the functionalised gelatin of step f).

An alternative method of preparing a carboxylic acid-functionalised -gelatin -gelatin as disclosed herein comprises the following steps:
a) modifying gelatin by reacting gelatin with a reagent containing an N-hydroxysuccinimide ester of the carboxylic acid moiety R-(CH₂)ₙ-COOH wherein n is an integer from 1 to 10 (R-(CH₂)ₙ-COO-NHS) in a reaction medium;
b1) lowering the pH of the reaction medium to a value between 4.0 and 9.0, preferably between 4.0 and 6.0, more preferably between 4.0 and 6.0, even more preferably between 4.5 and 5.5;
c) adding 0.01 - 1.5 w/w% of a micelle-forming surfactant to the reaction medium; b2). lowering the pH of the reaction medium to a value between 2.0 and 4.0, preferably between 2.5 and 3.5, more preferably between 3.0 and 3.5; ;
d) contacting the medium of step b2) with a solid adsorbent;
e). separating the solid adsorbent of step d) from the medium; and
f). recovering the medium comprising the functionalised gelatin.
g) optionally drying the medium comprising the carboxylic acid-functionalised - gelatin -gelatin of step f).

Any type of gelatin, e.g. type A or type B gelatin, of e.g. bovine, porcine, poultry or fish origin, can be used in the methods described herein. In embodiments, type A gelatin is used. In other embodiments, type B gelatin is used.

Carboxylic acid functionalisation of gelatin with N-hydroxy-succinimidyl-activated carboxylic acids can be performed by reacting gelatin with the respective N-hydroxy-succinimidyl-activated carboxylic acids in a suitable buffer, e.g. in carbonate buffer at pH 9.0 or in phosphate-buffered saline (PBS), at a temperature of 50°C for 60-180 min such as 60 minutes or 120-180 minutes. The degree of carboxylic acid functionalisation of the gelatin can be tailored by varying the N-hydroxy-succinimidyl-activated carboxylic acid reagent-to-gelatin ratio as described in Shirahama et al. (2016). During the reaction, carboxylic acid -gelatin are formed as well as free carboxylic acid.

In embodiments, the N-hydroxy-succinimidyl--activated carboxylic acid used in the functionalisation of the gelatin of the present invention is a N-hydroxy-succinimidyl- (NHS)-activated carboxylic acid having the formula R-(CH₂)ₙ-COO-NHS wherein n is an integer form 1 to 10, preferablt in the essential absence of 1-ethyl-3-(3-dimethylaminopropyl)-carbodiimide hydrochloride (EDC)

In embodiments, in the N-hydroxy-succinimidyl- (NHS)-activated carboxylic acid used in the functionalisation of the gelatin, R is selected from the group consisting of 4-phenol, 5-norbornenyl or SH. In embodiments, n is from 1 to 10, preferably from 1 to 5, preferably 2 or 3. In preferred embodiments, in the N-hydroxy-succinimidyl- (NHS)-activated carboxylic acid used in the functionalisation of the gelatin, the carboxylic acid moiety is one or more of 3-(4-hydroxyphenyl)-propionic acid, 3-(SH)-propionic acid, and 2-(5-norbornenyl)-acetic acid, preferably 3-(4-hydroxyphenyl)-propionic acid. In preferred embodiments, the N-hydroxy-succinimidyl- (NHS)-activated carboxylic acid used in the functionalisation of the gelatin, is selected form the group consisting of N-hydroxy-succinimidyl-3-(4-hydroxyphenyl)-propionate, N-hydroxy-succinimidyl-3-(SH)-propionate, and N-hydroxy-succinimidyl-2-(5-norbornenyl)-acetyl preferably N-hydroxy-succinimidyl-3-(4-hydroxyphenyl)-propionate.

The pH of the reaction medium may be lowered before adding the micelle-forming surfactant to a value between 2.0 and 5.0, preferably between 2.0 and 4.0, more preferably between 3.0 and 4.0 such as a value of about 3.5 or between 2.0 and 3.5 such as a value of about 3.0, even more preferably between 3.0 and 3.5 (step b) in the methods described herein). Alternatively, the pH of the reaction medium may be lowered to a value between 4.0 and 9.0, preferably between 4.0 and 6.0, more preferably between 4.0 and 6.0, even more preferably between 4.5 and 5.5 before adding the micelle-forming surfactant (step b1) in the methods described herein), and may be further lowered to a value between 2.0 and 4.0, more preferably between 3.0 and 4.0 such as a value of about 3.5 or between 2.0 and 3.5 such as a value of about 3.0, even more preferably between 3.0 and 3.5, after adding the micelle-forming surfactant and before contacting the medium with the solid adsorbent (step b2) in the methods described herein). Advantageously, when performing the next method steps at low pH, LPS, pyrogens and carboxylic acid are removed more efficiently.

In the methods described herein, a micelle-forming surfactant is added to the reaction medium. A micelle-forming surfactant is capable of forming micelles (soluble aggregates) in solution. Micelle-forming surfactants are known in the art. The micelle-forming surfactant can be an ionic surfactant, such as a cationic or anionic surfactant. Preferably, the surfactant is non-ionic surfactant as such a surfactant tends to form micelles at lower concentration as compared to ionic surfactants. Further, ionic surfactants may interact with the gelatin by ionic bonds, and are more difficult to remove.

Preferably, the micelle-forming non-ionic surfactant is an ethoxylated surfactant, preferably an alkylphenol ethoxylate, the alkylphenol ethoxylate preferably being represented by the formula CₓH₂ₓ₋₁-C₆H₄-O-(C₂H₄O)nH, wherein x is 4 -12 and n is 7.5 - 14, X preferably being 8 and n preferably being 8-13, more preferably 8.5-12.5, most preferably 9-12, in particular Triton X-100, Triton X-102, or mixtures thereof. The Triton X-series of nonionic surfactants are prepared by the reaction of octylphenol with ethylene oxide. The products are of the type commonly described as alkylaryl polyether alcohols and have the following structural formula: C₈H₁₇ C₆H₅(CH₂CH₂O)_{9.7} (Triton X-100) and C₉H₁₉ C₆H₅(CH₂CH₂O)12.3 (Triton X-102). Other non-ionic surfactants that are suitable comprise nonylphenoxypolyethoxyethanols C₁₅H₂₄O(C₂H₄O)ₙ, n being 3-40, such as nonoxynol-4,nonoxynol-15 and nonoxynol-30, or polyethylene glycol sorbitan monoesters of C₁₂-C₁₈ fatty acids, such as TWEEN. CHAPSO (3-([3- cholamidopropyl]dimethyl ammonio)-2-hydroxyl-1 - propanesulfonate is another suitable non-ionic surfactant.

As a result of adding a micelle-forming surfactant, LPS is monomerised and said monomers are believed to interact with the surfactant, forming micelle complexes comprising surfactant and LPS. In order to allow for efficient removal of the LPS, and preferably also of other pyrogens, in particular pyrogens from Gram+ bacteria and pyrogens from flagellated bacteria, more particularly pyrogens from Gram+ bacteria, the weight ratio of gelatin, in particular carboxylic acid -gelatin, to added surfactant, in particular non-ionic surfactant, is preferably 2000 : 1 or less, more preferably 500 : 1 or less, even more preferably 250 : 1 or less, most preferably 50 : 1 or less. Indeed, at higher weight ratios, i.e. where there is relatively more gelatin, not all LPS will be bound by the surfactant. Preferably, the surfactant is added to the reaction medium at a concentration of 0.01 - 1.5 w/w%, preferably 0.015 - 1.0 w/w%, more preferably 0.020-0.50 w/w%.

In step d) of the methods described herein, the medium of step c) or the medium of step b2), which may contain soluble aggregates comprising surfactant, LPS and monomers thereof and/or other pyrogens, in particular pyrogens from Gram+ bacteria and pyrogens from flagellated bacteria, more particularly pyrogens from Gram+ bacteria, is contacted with a (solid) adsorbant. In addition to binding the surfactant, and preferably also LPS, the adsorbent also preferably binds other pyrogens, in particular pyrogens from Gram+ bacteria, pyrogens from flagellated bacteria, single-stranded viral RNA, and bacterial DNA rich in unmethylated CpG motifs, and HPA.

The solid adsorbent can be any suitable adsorbent, capable of binding the surfactant, and preferably also LPS, other pyrogens and carboxylic acid, known to the skilled person, such as a hydrophobic adsorbent. The adsorbent is preferably insoluble, and suitable adsorbents comprises clays, such as (activated) diatomaceous earth or clays, phyllosilicates, such as aluminium phyllosilicate, smectite minerals and hydrophobic adsorbents, such as activated carbon, for example Norit SX Plus or Norit ROX 0.8 (Cabot, the Netherlands), or 3M ZetaCarbon filter cartridges, e.g. such as of the type R55S or R30L3S (3M, USA). Also mixtures of one or more adsorbents can be applied. In preferred embodiments, the solid adsorbent is activated carbon.

Contacting the medium with a solid adsorbent can be done e.g. by adding particulate adsorbent to the medium, or by passing the medium through a filter element comprising the said adsorbent or over a column stacked with the adsorbent, or by incubating the medium with a carrier having the adsorbent present on the outer surface thereof.

The solid adsorbent is preferably added to the medium in a weight ratio to the surfactant of at least 2.5 : 1 , more preferably of at least 3.0 : 1 , most preferably of at least 3.5 : 1. The solid adsorbent is preferably added to the medium in a concentration of 0.1 - 3 w/w%, preferably of 0.5 - 1 w/w%. In case filter elements or systems are used, it may be preferred to use similar amounts of adsorbent in the filter system.

The contacting step is performed for a sufficient time to allow proper adsorption of the surfactant, resulting in removal of the surfactant and the LPS and/or other pyrogens, in particular pyrogens from Gram+ bacteria and pyrogens from flagellated bacteria, more particularly pyrogens from Gram+ bacteria, which are bound to the surfactant, and preferably also to allow adsorption of the HPA, LPS and other pyrogens, in particular pyrogens from Gram+ bacteria, pyrogens from flagellated bacteria, single-stranded viral RNA, and bacterial DNA rich in unmethylated CpG motifs, and methacrylic acid, which are bound to the adsorbent. Preferably, the adsorbent is contacted with the (aqueous) medium for 5 minutes to 1 hour, more preferably for 10 - 30 minutes.

In the next step (e)) of the methods described herein, the solid adsorbent is removed from the medium. The skilled person is aware of suitable ways to bring an aqueous medium in contact with a solid adsorbent and to separate the adsorbent from the medium. The said separation can e.g. comprise centrifugation or filtration in case the adsorbent is added as a particulate to the medium, where filtration is preferred in view of industrial applicability. For example, the solid adsorbent can be added to the medium (of step c) or step b2) and, after allowing the surfactant and preferably also the LPS, other pyrogens and carboxylic acid to bind to the adsorbent, the adsorbent can be removed e.g. by filtration, sedimentation or centrifugation and the like. In another example, the solid adsorbent can be present in a filter, and the medium (of step c) or step b2) of the methods described herein) is passed through the said filter, or through a series of such filters, while the filters can optionally be washed in order to optimize the yield of the filtrate. This way, steps d), e) and f) of the herein described methods can be combined in a single filtration step Following the separation of the solid adsorbent, the medium comprising the carboxylic acid-gelatin is recovered.

Preferably steps c)-f) of the methods described herein are performed at a temperature below the cloud point of the surfactant used. As used herein, the term "cloud point" refers to the temperature at which the surfactant forms insoluble aggregates in the medium. Said temperature depends on the conditions of the medium, such as salt concentration. When no specific conditions are given, the cloud point is defined herein as the temperature where a 1 w/w% aqueous solution forms insoluble aggregates. So, if the temperature is described to be below the cloud point of a surfactant, said temperature is 68 - 69 °C (i.e. for a 1 w/w% Triton X- 100 solution), but in case of a 16-25 w/w% NaCl solution, said cloud point is room temperature. The cloud point can conveniently be determined under the given circumstances, by determining the light absorbance of the solution at 620 nm without addition of the surfactant, and check whether the absorbance increases when the envisaged amount of surfactant is added. Above the cloud point, the absorbance is increased.

In embodiments, steps c)-f) are performed at a temperature of 65 °C or less, more preferably of 62 °C or less, even more preferably of 60 °C or less. In embodiments, steps c)-f) are performed at a temperature between 30 °C and 65 °C, preferably between 30 °C and 60 °C, more preferably between 30 °C and 50 °C or between 30 °C and 40 °C, even more preferably between 30 °C and 35°C.

In embodiments, the pH of the medium is between 2.0 and 5.0, preferably between 2.0 and 4.0, more preferably between 3.0 and 4.0 such as at about 3.5 or between 2.0 and 3.5 such as at about 3.0, even more preferably between 3.0 and 3.5 throughout the method steps c)-f). At such pH, the temperature is preferably below 35°C, more preferably between 30°C and 35°C, such as at about 30°C.

In other embodiments, , the pH of the medium is between 2.0 and 5.0, preferably between 2.0 and 4.0, more preferably between 3.0 and 4.0 such as at about 3.5 or between 2.0 and 3.5 such as at about 3.0, even more preferably between 3.0 and 3.5 throughout the method steps d)-f). At such pH, the temperature is preferably below 35°C, more preferably between 30°C and 35°C, such as at about 30°C.

Following the recovery of the medium comprising the carboxylic acid-gelatin, the methods may further comprise a step of increasing the pH of the medium to between 3.5 and 9.0, preferably between 4.0 and 8.0, more preferably between 5.0 and 7.0. As noted before, the methods described herein results in carboxylix acid-gelatin with low LPS content, in particular an LPS content of less than 100 EU/g, more preferably less than 50 EU/g, even more preferably less than 20 EU/g, even more preferably less than 10 EU/g, even more preferably less than 5 EU/g, even more preferably less than 2 EU/g, most preferably less than 1 EU/g. In particular, the carboxylic acid-gelatin -gelatin comprises at least 50 times less LPS, preferably at least 100 times, more preferably at least 150 times, even more 5 preferably at least 200 times and most preferably at least 250 times less LPS as compared to the LPS content of the gelatin used as starting material of step a). The LPS count can be determined on the recovered medium using e.g. the LAL assay as described elsewhere herein.

The obtained carboxylic acid-gelatin is further characterized by a low content of nonendotoxin pyrogens, in particular a low content of pyrogens from Gram+ bacteria, pyrogens from flagellated bacteria, single-stranded viral RNA, endotoxin from Gram-bacteria and bacterial DNA rich in unmethylated CpG motifs, more particularly a low content of pyrogens from Gram+ bacteria and from flagellated bacteria, even more particularly a low content of pyrogens from Gram+bacteria. In particular, the carboxylic acid-gelatin comprises at least 10 times less pyrogens from Gram+ bacteria, preferably at least 20 times less, more preferably at least 50 times, even more preferably at least 100 times, at least 150 times, at least 200 times or at least 250 times less, as compared to the content of pyrogens from Gram+ bacteria of the gelatin used as starting material of step a). In particular, the carboxylic acid-gelatin comprises at least 10 times less pyrogens from flagellated bacteria, preferably at least 20 times less, more preferably at least 50 times, even more preferably at least 100 times, at least 150 times, at least 200 times or at least 250 times less, as compared to the content of pyrogens from flagellated bacteria of the gelatin used as starting material of step a). In particular, the carboxylic acid-gelatin comprises at least 10 times less bacterial DNA rich in unmethylated CpG motifs, preferably at least 20 times less, more preferably at least 50 times, even more preferably at least 100 times, at least 150 times, at least 200 times or at least 250 times less, as compared to the content of less bacterial DNA rich in unmethylated CpG motifs of the gelatin used as starting material of step a). less single-stranded viral RNA.

Further, the methods described herein result in carboxylic acid-modified gelatin with low free (non-gelatin bound) carboxylic acid content, in particular less than 100 ppm, preferably less than 50 ppm, more preferably less than 30 ppm of carboxylic acid. The low carboxylic acid functionalized gelatin was dissolved in 50 mM phosphate buffer, pH 9.5 for determining free (non-gelatin bound) 3-(4-hydroxyphenyl)-propionic acid content.
, preferably as determined on a sample of the carboxylic acid -gelatin dissolved in water, of less than 150 ppm, preferably less than 100 ppm of carboxylic acid as determined on a sample of the carboxylic acid -gelatin dissolved in 50 mM phosphate buffer, pH 9.5.

Thus, the present methods provide a purified carboxylic acid-gelatin in a single round of the (6 or 7) steps a)-f) described above.

As the methods described herein result in carboxylic acid-gelatin with low carboxylic acid content without the need of a dialysis step, the methods are preferably free of a dialysis step. Such dialysis step is in general time-consuming, and therefore, the present methods are more efficient, especially for large scale production of a purified carboxylic acid-gelatin.

In embodiments, the methods further comprise a step of drying the recovered medium comprising the carboxylic acid gelatin, optionally after having increased the pH of the medium, e. g. by freeze-drying to a white porous foam (Van Den Bulcke et al., 2000., Biomacromolecules, 1:31-38).

The present invention will now be further illustrated by means of the following non-limiting examples.

### EXAMPLES

### Example 1: 3-(4-hydroxyphenyl)-propionate functionalized-gelatin preparation method and properties of the 3-(4-hydroxyphenyl)-propionate functionalized -gelatin

### Materials and methods

15-20 % gelatin (pig skin, type A, Bloom 200) solution in 1.25 M carbonate buffer at pH 9.0 was reacted with N-hydroxysuccinimide-3-(4-hydroxyphenyl)-propionate in a 1:1 ratio to the total available amino groups in the gelatin for 120-180 minutes at 50°C. During the reaction, 3-(4-hydroxyphenyl)-propionic acid (PA) was formed. The pH of the reaction medium was lowered to pH 3.5. HCl was used to remove the carbonate. 1.12% (on weight of gelatin) Triton-X100 was added to the medium and the medium was maintained under agitation during 1 hour. The medium was then filtered over a column packed with active carbon. The other N-hydroxysuccinimide esters of carboxylic acids were reacted analogously.

### Determination of LPS content

LPS content was determined using the EndoZyme II assay kit (Hyglos) according to the manufacturer's instructions. Briefly, functionalised gelatin solutions in ultrapure water were prepared at 2.50% (w/w) and 10x, 20x, 50x, 100x, etc. dilutions depending on the expected LPS content. 100 µL of each sample was added into a well of a multi-well plate and mixed with 100 µL of the assay reagent prepared according to the manufacturer's instructions. Fluorescence intensity of the resulting mixtures was monitored in a microplate fluorescence reader (BopTek; excitation/emission = 380 nm/455 nm). A dilution series of an endotoxin standard (E. coli 055:B5) reconstituted in endotoxin free water at 50 EU/mL was used as calibration standard; and endotoxin free water was used as blank determination of 4-hydroxyphenyl-propionic acid content 8% (w/w) solutions of the functionalised gelatin (GelDAT) samples in water or 50 mM phosphate buffer, pH 9.5 were made. The samples were allowed to swell for 15 min at room temperature, and were then put at 50°C for 30 min until the samples were visually completely dissolved. 0.5 mL of the sample solutions were added to 10-kDa Amicon Ultra Centrifugal Filters (Milipore) and put at 50°C (oven) for 10 min, after which they were centrifuged at 12000 xg for 30 min at 40°C. Afterwards, the filtrates were collected for HPLC analysis. A dilution series of 4-hydroxyphenyl-propionic acid ranging from 0.1 ppm to 100 ppm was made by preparing a 1% (w/w) of 4-hydroxyphenyl-propionic acid in water or 50 mM phosphate buffer, pH 9.5 and making 2-fold dilutions in water or 50 mM phosphate buffer, pH 9.5, and used as standard.

### Determination of DNA content

A stock concentration of 0.1 mg/mL salmon testes dsDNA standard was prepared in TE buffer (10mM Tris-HCl with 1mM EDTA in water, pH = 8) and diluted further to a stock concentration of 10 µg/mL. Quantification standards ranging from 10 µg/ml to 0 µg/ml in TE buffer. 10% (w/w) gelatin samples were prepared in TE buffer; the samples were allowed to swell for 30 min and dissolved at 40°C using a water bath. 95 µl of a master mix containing 94 µL TE buffer and 1 µL SYBR Green (100x), and 5 µl of a sample or standard were added in the wells of a black well fluorescent 96-well plate and mixed by pipetting up and down. The 96-well plate was covered and incubated at 37°C and 700 rpm for 30 min. Fluorescence was measured at Ex535nm/Em617nm using a Synergy^{™} Mx fluorometer (BioTek) at 25°C according to the manufacturer's instructions.

### Example 2: Comparison of functionalized gelatins prepared by a method according to an embodiment of the invention and a method based on dialysis.

### Material and methods

Gelatin was modified according to the method described herein. The start gelatin was contaminated with 2229 endotoxin units (EU) per gram of gelatin (Hyglos endozyme). 3-(4-hydroxyphenyl)-propionic acid residues and LPS contamination were measured as described in Example 1 in ppm, and EU per gram of gelatin, respectively. The functionalized gelatin was dissolved in 50 mM phosphate buffer, pH 9.5 for determining 3-(4-hydroxyphenyl)-propionic acid content. In the comparative method, 3-(4-hydroxyphenyl)-propionic acid by-product was removed from the functionalized gelatin solutions by dialysis against ultrapure water (MilliQ, Merck Millipore). Twenty milliliter aliquots of the 3-(4-hydroxyphenyl)-propionic acid solutions were transferred to 5 dialysis tubes (Dialysis tubing cellulose membrane, MWCO 14 kDa, cat. N°: D9527-100FT, Sigma-Aldrich) and each of these were submerged into 2.5 liter of water. Dialysis proceeded at 40°C, and the water was exchanged every 24 h. At days T1=1, T2=2, T3=3, T4=4, and T5=7, one tube was removed and its 3-(4-hydroxyphenyl)-propionic acid and LPS content were measured as described in Example 1. The functionalized gelatin was dissolved in 50 mM phosphate buffer, pH 9.5 for determining 3-(4-hydroxyphenyl)-propionic acid content. In the method according to an embodiment of the invention, five 40 ml aliquots of the functionalized gelatin solutions were pH adjusted by adding diluted HCl to a pH of 2.0, 2.5, 3.0, 3.5, and 4.0 (all measured at 40°C) and 0,1 % (on gelatin content) of Triton-X100 was added to each aliquot. Each aliquot was then divided in two, with one of those serving as a negative control on the effect of the pH reduction, and Triton-X100 addition on the methacrylic acid content and the LPS contamination. Five gram of Norit active carbon powder (S268, active carbon Norit SX plus 8013- 1) was added to each of the experimental tubes, and these tubes were incubated on rotary shaker for 1 h at 40 °C. Next, these tubes were centrifuged at 2000 rpm for 30 minutes, and the supernatant was filtered (0,45 µm) and analyzed as described in Example 1 for its 3-(4-hydroxyphenyl)-propionic acid and LPS content. The functionalized gelatin was dissolved in 50 mM phosphate buffer, pH 9.5 for determining free (non-gelatin bound) 3-(4-hydroxyphenyl)-propionic acid content.

### Results

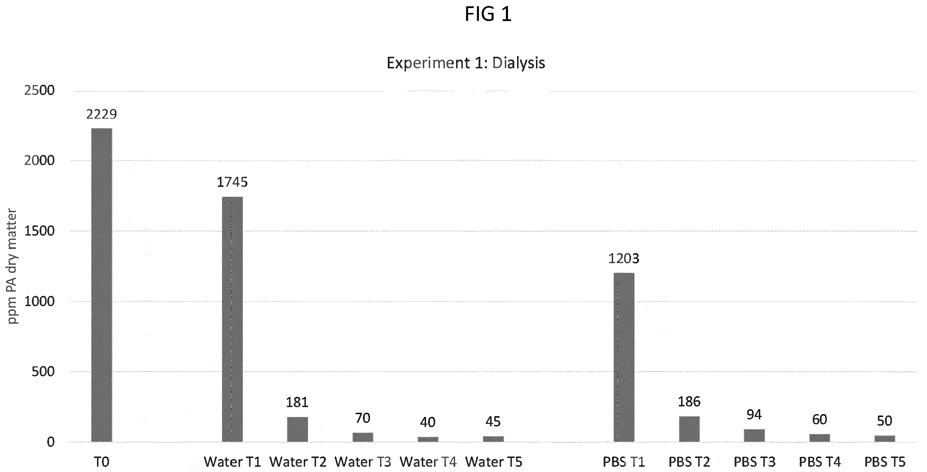

Table 1 shows the 3-(4-hydroxyphenyl)-propionic acid (PA) content (ppm) and LPS content (EU/g) of purified functionalized gelatin obtained by the method disclosed herein elsewhere. Figure 1 is the graphic representation.

### Purification via dialysis:

| Table 1 Figure 1 | | **Water** | | | | | **PBS** | | | | |
|---|---|---|---|---|---|---|---|---|---|---|---|
| sample | T0 | T1 | T2 | T3 | T4 | T5 | T1 | T2 | T3 | T4 | T5 |
| LPS (EU/g) | 5291 | 5137 | 5415 | 5596 | 5530 | 5607 | 5291 | 5344 | 5318 | 5383 | 5421 |
| Ppm PA on dry product | 2229 | 1745 | 181 | 70 | 40 | 45 | 1203 | 186 | 94 | 60 | 50 |
| Remark | T0 | | | | | End point | | | | | End point |
| Reduction of PA % | 0 | 22 | 92 | 97 | | 98 | 46 | 92 | 96 | 97 | 98 |

### Purification via micelle-forming and filtration

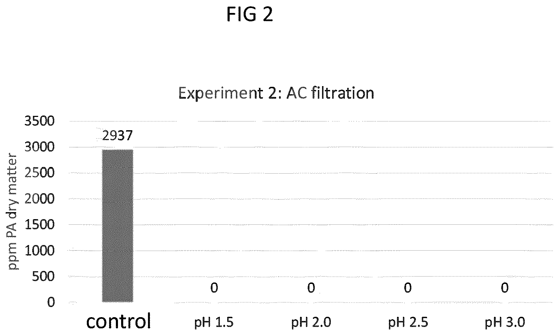

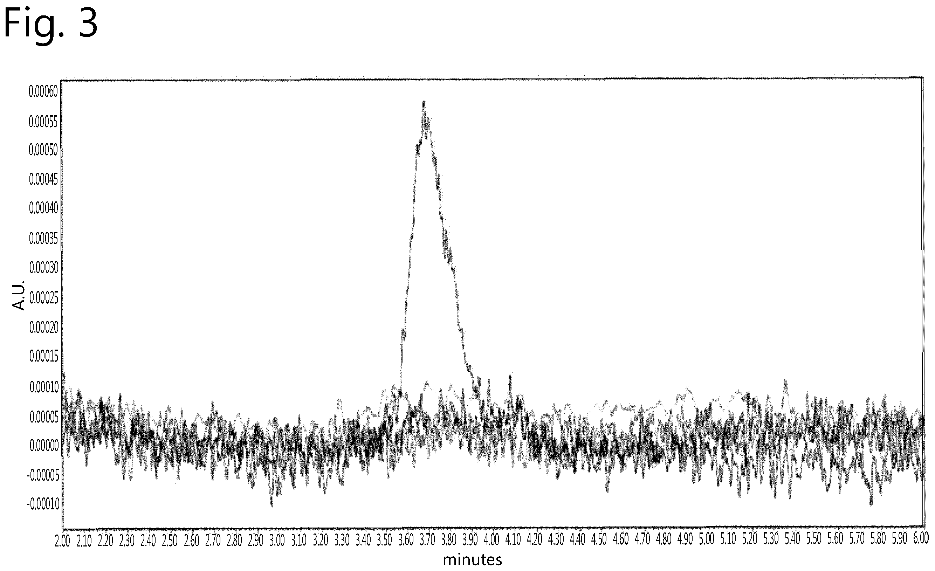

Table 2. shows the 3-(4-hydroxyphenyl)-propionic acid content (PA, ppm) and LPS content (EU/g) of purified functionalized gelatin obtained by a method based on dialysis or by a method according to an embodiment of the invention. Figure 2 is the graphic representation. Figure 3 shows the HPLC.

| Table 2 | Control | pH 1.5 | pH 2.0 | pH 2.5 | pH 3.0 |
|---|---|---|---|---|---|
| LPS (EU/g) | 2791 | 0.9 | 1.3 | 3.1 | 2.6 |
| ppm PA on dry product | 2937 | <1ppm* | <1ppm* | <1ppm* | <1ppm* |
| Reduction % | | 100 | 100 | 100 | 100 |

| | | | | | |
|---|---|---|---|---|---|
| *Below level of quantification | | | | | |

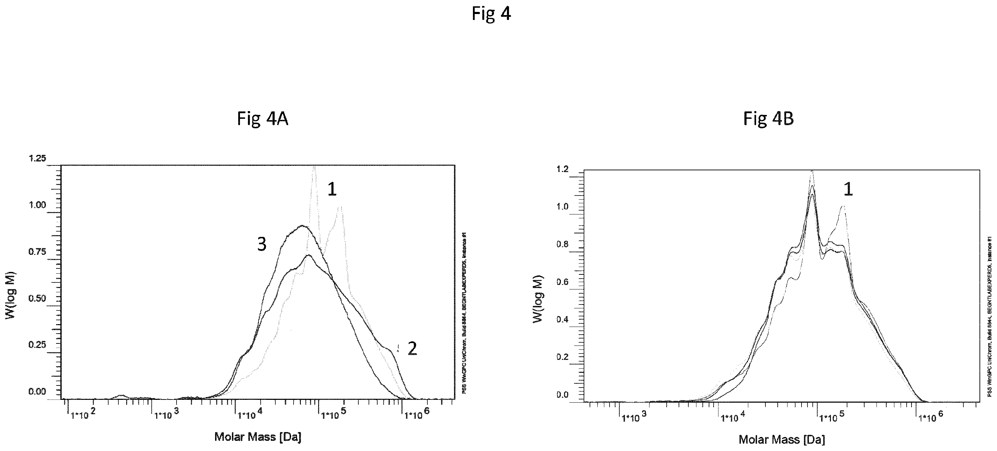

Figure 4 shows in figure 4A the MW distribution for the starting gelatin (1) and the results of a modification via a route using an in situ mixture of NHS, HPA and EDC followed by purification using one step dialysis (2) and two-step dialysis (3). The modified gelatin has a broader Mw distribution compared to the starting gelatin, indicative of undesired crosslinking.

Figure 4B shows in figure 4A the MW distribution for the same starting gelatin (1) and the results of a modification via a route using an NHS-HPA in essential absence of EDC, followed by purification using a purification based on micelle formation. The modified gelatin has an Mw distribution that is nearly identical to the starting gelatin, indicative of successful derivatization in essential absence of undesired crosslinking.

The derivatization with NHS-HPA in absence of EDC compared to a mixture of NHS, HPA and EDC leads to a product that has a different Mw profile, closer to the original gelatin. The purification through micelles formation leads to a product that is low in LPS as well as low in HPA and hence to an improved quality.

## Claims

1. Gelatin functionalised via an amide bond with a carboxylic acid moiety R-(CH₂)ₙ-COOH,
wherein R is selected from the group consisting of 4-phenol, norbornenyl or SH and
wherein n is an integer from 1 to 10, and
wherein the functionalised gelatin has a lipopolysaccharide content of less than 100 EU/g, preferably less than 50 EU/g, more preferably less than 20 EU/g, even more preferably less than 10 EU/g, even more preferably less than 5 EU/g, even more preferably less than 2 EU/g, most preferably less than 1 EU/g, and
wherein the functionalised gelatin contains an amount of free carboxylic acid R-(CH₂)ₙ-COOH of than less than 100 ppm, wherein the amount of free carboxylic acid R-(CH₂)ₙ-COOH is determined by high-performance liquid chromatography on a filtrate obtained by dissolving gelatin in 50 mM phosphate buffer, pH 9.5, and ultrafiltration of the dissolved sample.

2. Gelatin according to claim 1 , wherein n is an integer from 1 to 5, preferably 2 or 3.

3. Gelatin according to claims 1 or 2, wherein the carboxylic acid moiety is one or more of 3- (4-hydroxyphenyl)-propionic acid, 3-(SH)-propionic acid, and 2-(5-norbornenyl)-acetic acid, preferably 3-(4-hydroxyphenyl)-propionic acid.

4. Gelatin according to claims 1-3, wherein the functionalised gelatin has a degree of modification with the carboxylic acid moiety of between 5% and 100%, preferably between 25% and 100%, more preferably between 50% and 100%, even more preferably between 75 and 100%.

5. Gelatin according to claims 1-4, wherein the functionalised gelatin contains an amount of free carboxylic acid R-(CH₂)ₙ-COOH of less than 50 ppm, more preferably less than 20 ppm, even more preferably less than 10 ppm, even more preferably less than 5 ppm, even more preferably less than 2 ppm, most preferably less than 1 ppm.

6. Gelatin according to claim 5, wherein the functionalised gelatin contains an amount of free carboxylic acid R-(CH₂)ₙ-COOH of than less than 30 ppm.

7. Gelatin according to the above claims, wherein the functionalised gelatin is a type A gelatin.

8. Gelatin according to the above claims, wherein the functionalised gelatin is further modified with an (meth)acrylate group or moiety, an acetyl group or moiety, a phenol group or moiety, a thiol group or moiety, a norbornene group or moiety, a tetrazine group or moiety, an azide group or moiety, a furan group or moiety, an allyl group or moiety, a maleimide group or moiety or any combination thereof.

9. Method for providing a functionalised gelatin according to any one of claims 1 to 8, the method comprising the steps of
a. modifying gelatin by reacting gelatin with a reagent containing an *N-*hydroxysuccinimide ester of the carboxylic acid moiety R-(CH₂)ₙ-COOH wherein n is an integer from 1 to 10 (R-(CH₂)ₙ-COO-NHS) in a reaction medium;
b. lowering the pH of the reaction medium to a value between 2.0 and 3.5;
c. adding 0.01- 1.5 w/w% of a micelle-forming surfactant to the acidic reaction medium;
d. contacting the micelle-containing medium with an adsorbent, preferably activated carbon;
e. separating the adsorbent from the medium;
f. recovering the medium comprising the functionalised gelatin,
wherein R is selected from the group consisting of 4-phenol, norbornenyl and SH.

10. Method for preparing a functionalised gelatin according to any one of claims 1 to 8, comprising the steps of:
a) modifying gelatin by reacting gelatin with a reagent containing an N-hydroxysuccinimide ester of the carboxylic acid moiety R-(CH₂)ₙ-COOH wherein n is an integer from 1 to 10 (R-(CH₂)ₙ-COO-NHS) in a reaction medium;
b1). lowering the pH of the reaction medium to a value between 4.0 and 9.0, preferably between 4.0 and 6.0, more preferably between 4.0 and 6.0, even more preferably between 4.5 and 5.5;
c) adding 0.01 - 1.5 w/w% of a micelle-forming surfactant to the reaction medium;
b2). lowering the pH of the reaction medium to a value between 2.0 and 3.5;
d). contacting the medium of step b2) with a solid adsorbent;
e). separating the solid adsorbent of step d) from the medium; and
f). recovering the medium comprising the functionalised gelatin,
wherein R is selected from the group consisting of 4-phenol, norbornenyl and SH.

11. Method according to claim 9 or 10, wherein n is an integer from 1 to 5, preferably 2 or 3.

12. Method according to claims 9-11, wherein the reagent containing an N-hydroxysuccinimide ester of the carboxylic acid moiety R-(CH₂)ₙ-COOH wherein n is an integer from 1 to 10, is R-(CH₂)ₙ-COO-NHS.

13. Method according to claims 9-12, wherein the carboxylic acid moiety is one or more of 3-(4-hydroxyphenyl)-propionic acid, 3-(SH)-propionic acid, and 2-(5-norbornenyl)-acetic acid, preferably 3-(4-hydroxyphenyl)-propionic acid.

14. Method according to claims 9-13, wherein the primary amine groups of gelatin are modified with the carboxylic acid moiety.

15. Method according to claims 9-14, wherein a primary amine group of the functionalised gelatine is coupled to the carboxylic acid moiety via an amide bond.

16. Method according to claims 9-15, wherein the method does not comprise a dialysis step.

17. Method according to any one of claims 9-16, wherein said micelle-forming surfactant comprises a non-ionic surfactant, preferably wherein the surfactant is Triton X-100 or Triton X- 102, or mixtures thereof.

18. Method according to claims 9-17, wherein the functionalised gelatin has a lipopolysaccharide content of less than 100 EU/g, preferably less than 50 EU/g, more preferably less than 20 EU/g, even more preferably less than 10 EU/g, even more preferably less than 5 EU/g, even more preferably less than 2 EU/g, most preferably less than 1 EU/g.

19. Method according to claims 9-18, wherein the functionalised gelatin has a degree of modification with the carboxylic acid moiety of between 5% and 100%, preferably between 25% and 100%, more preferably between 50% and 100%, even more preferably between 75 and 100%.

20. Method according to claims 9-19, wherein the functionalised gelatin contains an amount of free carboxylic acid R-(CH₂)ₙ-COOH of than less than 100 ppm, preferably less than 50 ppm, more preferably less than 20 ppm, even more preferably less than 10 ppm, even more preferably less than 5 ppm, even more preferably less than 2 ppm, most preferably less than 1 ppm,
wherein the amount of free carboxylic acid R-(CH₂)ₙ-COOH is determined by high-performance liquid chromatography on a filtrate obtained by dissolving gelatin in 50 mM phosphate buffer, pH 9.5, and ultrafiltration of the dissolved sample.

21. Method according to claims 9-20, wherein functionalised gelatin is further modified with an (meth)acrylate group or moiety, an acetyl group or moiety, a phenol group or moiety, a thiol group or moiety, a norbornene group or moiety, a tetrazine group or moiety, an azide group or moiety, a furan group or moiety, an allyl group or moiety, a maleimide group or moiety or any combination thereof.

22. Method according to any one of claims 9-21, comprising a step of drying the medium comprising the functonalised gelatin of step f).

23. Hydrogel comprising a functionalised gelatin as defined in any of claims 1-8 and a cross-linking agent.

24. Film comprising a hydrogel according to claim 23 or a functionalised gelatin according to claims 1-8.

## Patentansprüche

1. Gelatine, funktionalisiert über eine Amidbindung mit einer Carbonsäuregruppierung R-(CH₂)ₙ-COOH, wobei R ausgewählt ist aus der Gruppe bestehend aus 4-Phenol, Norbornenyl oder SH und wobei n für eine ganze Zahl von 1 bis 10 steht und
wobei die funktionalisierte Gelatine einen Lipopolysaccharidgehalt kleiner 100 EU/g, bevorzugt kleiner 50 EU/g, bevorzugter kleiner 20 EU/g, noch bevorzugter kleiner 10 EU/g, noch bevorzugter kleiner 5 EU/g, noch bevorzugter kleiner 2 EU/g, am meisten bevorzugt kleiner 1 EU/g, aufweist und
wobei die funktionalisierte Gelatine eine Menge an freier Carbonsäure R-(CH₂)ₙ-COOH von weniger als 100 ppm enthält, wobei die Menge an freier Carbonsäure R-(CH₂)ₙ-COOH mit Hochleistungsflüssigkeitschromatographie an einem durch Lösen von Gelatine in 50 mM Phosphatpuffer, pH 9,5, und Ultrafiltration der gelösten Probe erhaltenen Filtrat bestimmt wird.

2. Gelatine nach Anspruch 1, wobei n für eine ganze Zahl von 1 bis 5, bevorzugt 2 oder 3, steht.

3. Gelatine nach Anspruch 1 oder 2, wobei es sich bei der Carbonsäuregruppierung um eine oder mehrere von 3-(4-Hydroxyphenyl)propionsäure, 3-(SH)-Propionsäure und 2-(5-Norbornenyl)essigsäure, bevorzugt 3-(4-Hydroxyphenyl)propionsäure, handelt.

4. Gelatine nach Anspruch 1 bis 3, wobei die funktionalisierte Gelatine einen Grad der Modifikation mit der Carbonsäuregruppierung zwischen 5 % und 100 %, bevorzugt zwischen 25 % und 100 %, bevorzugter zwischen 50 % und 100 %, noch bevorzugter zwischen 75 und 100 %, aufweist.

5. Gelatine nach Anspruch 1 bis 4, wobei die funktionalisierte Gelatine eine Menge an freier Carbonsäure R-(CH₂)ₙ-COOH von weniger als 50 ppm, bevorzugter weniger als 20 ppm, noch bevorzugter weniger als 10 ppm, noch bevorzugter weniger als 5 ppm, noch bevorzugter weniger als 2 ppm, am meisten bevorzugt weniger als 1 ppm enthält.

6. Gelatine nach Anspruch 5, wobei die funktionalisierte Gelatine eine Menge an freier Carbonsäure R-(CH₂)ₙ-COOH von weniger als 30 ppm enthält.

7. Gelatine nach den obigen Ansprüchen, wobei es sich bei der funktionalisierten Gelatine um eine Gelatine vom Typ A handelt.

8. Gelatine nach den obigen Ansprüchen, wobei die funktionalisierte Gelatine weiter mit einer (Meth)acrylatgruppe oder -gruppierung, einer Acetylgruppe oder -gruppierung, einer Phenolgruppe oder -gruppierung, einer Thiolgruppe oder -gruppierung, einer Norbornengruppe oder -gruppierung, einer Tetrazingruppe oder -gruppierung, einer Azidgruppe oder -gruppierung, einer Furangruppe oder -gruppierung, einer Allylgruppe oder -gruppierung, einer Maleimidgruppe oder -gruppierung oder einer beliebigen Kombination davon modifiziert ist.

9. Verfahren zur Bereitstellung einer funktionalisierten Gelatine nach einem der Ansprüche 1 bis 8, wobei das Verfahren die folgenden Schritte umfasst:
a. Modifizieren von Gelatine durch Umsetzen von Gelatine mit einem Reagenz, das einen N-Hydroxysuccinimidester der Carbonsäuregruppierung R-(CH₂)ₙ-COOH, wobei n für eine ganze Zahl von 1 bis 10 steht, enthält (R-(CH₂)ₙ-COO-NHS), in einem Reaktionsmedium;
b. Senken des pH des Reaktionsmediums auf einen Wert zwischen 2,0 und 3,5;
c. Versetzen des sauren Reaktionsmediums mit 0,01-1,5 Gew.-% eines micellenbildenden Tensids;
d. Inkontaktbringen des micellenhaltigen Mediums mit einem Adsorptionsmittel, bevorzugt Aktivkohle;
e. Trennen des Adsorptionsmittels vom Medium;
f. Gewinnen des die funktionalisierte Gelatine umfassenden Mediums,
wobei R aus der Gruppe bestehend aus 4-Phenol, Norbornenyl und SH ausgewählt ist.

10. Verfahren zur Herstellung einer funktionalisierten Gelatine nach einem der Ansprüche 1 bis 8, umfassend die Schritte:
a) Modifizieren von Gelatine durch Umsetzen von Gelatine mit einem Reagenz, das einen N-Hydroxysuccinimidester der Carbonsäuregruppierung R-(CH₂)ₙ-COOH, wobei n für eine ganze Zahl von 1 bis 10 steht, enthält (R-(CH₂)ₙ-COO-NHS), in einem Reaktionsmedium;
b1). Senken des pH des Reaktionsmediums auf einen Wert zwischen 4,0 und 9,0, bevorzugt zwischen 4,0 und 6,0, bevorzugter zwischen 4,0 und 6,0, noch bevorzugter zwischen 4,5 und 5,5;
c) Versetzen des Reaktionsmediums mit 0,01-1,5 Gew.-% eines micellenbildenden Tensids;
b2). Senken des pH des Reaktionsmediums auf einen Wert zwischen 2,0 und 3,5;
d). Inkontaktbringen des Mediums aus Schritt b2) mit einem festen Adsorptionsmittel;
e). Trennen des festen Adsorptionsmittels aus Schritt d) vom Medium; und
f). Gewinnen des die funktionalisierte Gelatine umfassenden Mediums,
wobei R aus der Gruppe bestehend aus 4-Phenol, Norbornenyl und SH ausgewählt ist.

11. Verfahren nach Anspruch 9 oder 10, wobei n für eine ganze Zahl von 1 bis 5, bevorzugt 2 oder 3, steht.

12. Verfahren nach Anspruch 9 bis 11, wobei es sich bei dem Reagenz, das einen N-Hydroxysuccinimidester der Carbonsäuregruppierung R-(CH₂)ₙ-COOH, wobei n für eine ganze Zahl von 1 bis 10 steht, enthält, um R-(CH₂)ₙ-COO-NHS handelt.

13. Verfahren nach Anspruch 9 bis 12, wobei es sich bei der Carbonsäuregruppierung um eine oder mehrere von 3-(4-Hydroxyphenyl)propionsäure, 3-(SH)-Propionsäure und 2-(5-Norbornenyl)essigsäure, bevorzugt 3-(4-Hydroxyphenyl)propionsäure, handelt.

14. Verfahren nach Anspruch 9 bis 13, wobei die primären Amingruppen der Gelatine mit der Carbonsäuregruppierung modifiziert werden.

15. Verfahren nach Anspruch 9 bis 14, wobei eine primäre Amingruppe der funktionalisierten Gelatine über eine Amidbindung an die Carbonsäuregruppierung gekoppelt wird.

16. Verfahren nach Anspruch 9 bis 15, wobei das Verfahren keinen Dialyseschritt umfasst.

17. Verfahren nach einem der Ansprüche 9 bis 16, wobei das micellenbildende Tensid ein nichtionisches Tensid umfasst, vorzugsweise wobei es sich bei dem Tensid um Triton X-100 oder Triton X-102 oder Mischungen davon handelt.

18. Verfahren nach Anspruch 9-17, wobei die funktionalisierte Gelatine einen Lipopolysaccharidgehalt kleiner 100 EU/g, bevorzugt kleiner 50 EU/g, bevorzugter kleiner 20 EU/g, noch bevorzugter kleiner 10 EU/g, noch bevorzugter kleiner 5 EU/g, noch bevorzugter kleiner 2 EU/g, am meisten bevorzugt kleiner 1 EU/g, aufweist.

19. Verfahren nach Anspruch 9 bis 18, wobei die funktionalisierte Gelatine einen Grad der Modifikation mit der Carbonsäuregruppierung zwischen 5 % und 100 %, bevorzugt zwischen 25 % und 100 %, bevorzugter zwischen 50 % und 100 %, noch bevorzugter zwischen 75 und 100 %, aufweist.

20. Verfahren nach Anspruch 9 bis 19, wobei die funktionalisierte Gelatine eine Menge an freier Carbonsäure R-(CH₂)ₙ-COOH von weniger als 100 ppm, bevorzugt weniger als 50 ppm, bevorzugter weniger als 20 ppm, noch bevorzugter weniger als 10 ppm, noch bevorzugter weniger als 5 ppm, noch bevorzugter weniger als 2 ppm, am meisten bevorzugt weniger als 1 ppm enthält,
wobei die Menge an freier Carbonsäure R-(CH₂)ₙ-COOH mit Hochleistungsflüssigkeitschromatographie an einem durch Lösen von Gelatine in 50 mM Phosphatpuffer, pH 9,5, und Ultrafiltration der gelösten Probe erhaltenen Filtrat bestimmt wird.

21. Verfahren nach Anspruch 9 bis 20, wobei funktionalisierte Gelatine weiter mit einer (Meth)acrylatgruppe oder -gruppierung, einer Acetylgruppe oder - gruppierung, einer Phenolgruppe oder -gruppierung, einer Thiolgruppe oder - gruppierung, einer Norbornengruppe oder -gruppierung, einer Tetrazingruppe oder -gruppierung, einer Azidgruppe oder -gruppierung, einer Furangruppe oder -gruppierung, einer Allylgruppe oder -gruppierung, einer Maleimidgruppe oder -gruppierung oder einer beliebigen Kombination davon modifiziert ist.

22. Verfahren nach einem der Ansprüche 9 bis 21, umfassend einen Schritt, bei dem das Medium, das die funktionalisierte Gelatine aus Schritt f) umfasst, getrocknet wird.

23. Hydrogel, umfassend eine funktionalisierte Gelatine gemäß einem der Ansprüche 1 bis 8 und ein Vernetzungsmittel.

24. Folie, umfassend ein Hydrogel nach Anspruch 23 oder eine funktionalisierte Gelatine nach Anspruch 1 bis 8.

## Revendications

1. Gélatine fonctionnalisée via une liaison amide avec un groupement acide carboxylique R-(CH₂)ₙ-COOH, dans laquelle R est choisi dans le groupe constitué par 4-phénol, norbornényle ou SH et dans laquelle n est un entier de 1 à 10, et
dans laquelle la gélatine fonctionnalisée a une teneur en lipopolysaccharide inférieure à 100 UE/g, de préférence inférieure à 50 UE/g, plus préférablement inférieure à 20 UE/g, encore plus préférablement inférieure à 10 UE/g, encore plus préférablement inférieure à 5 UE/g, encore plus préférablement inférieure à 2 UE/g, de manière préférée entre toutes inférieure à 1 UE/g, et
dans laquelle la gélatine fonctionnalisée contient une quantité d'acide carboxylique libre R-(CH₂)ₙ-COOH inférieure à 100 ppm, la quantité d'acide carboxylique libre R-(CH₂)ₙ-COOH étant déterminée par chromatographie liquide haute performance sur un filtrat obtenu par dissolution de gélatine dans du tampon phosphate 50 mM, pH 9,5, et ultrafiltration de l'échantillon dissous.

2. Gélatine selon la revendication 1, dans laquelle n est un entier de 1 à 5, de préférence 2 ou 3.

3. Gélatine selon la revendication 1 ou 2, dans laquelle le groupement acide carboxylique est un ou plusieurs parmi acide 3-(4-hydroxyphényl)-propionique, acide 3-(SH)-propionique et acide 2-(5-norbornényl)-acétique, de préférence acide 3-(4-hydroxyphényl)-propionique.

4. Gélatine selon les revendications 1 à 3, dans laquelle la gélatine fonctionnalisée a un degré de modification avec le groupement acide carboxylique compris entre 5 % et 100 %, de préférence entre 25 % et 100 %, de préférence encore entre 50 % et 100 %, encore plus préférablement entre 75 et 100 %.

5. Gélatine selon les revendications 1 à 4, dans laquelle la gélatine fonctionnalisée contient une quantité d'acide carboxylique libre R-(CH₂)ₙ-COOH inférieure à 50 ppm, plus préférablement inférieure à 20 ppm, encore plus préférablement inférieure à 10 ppm, encore plus préférablement inférieure à 5 ppm, encore plus préférablement inférieure à 2 ppm, tout particulièrement préférablement inférieure à 1 ppm.

6. Gélatine selon la revendication 5, dans laquelle la gélatine fonctionnalisée contient une quantité d'acide carboxylique libre R-(CH₂)ₙ-COOH inférieure à 30 ppm.

7. Gélatine selon les revendications précédentes, dans laquelle la gélatine fonctionnalisée est une gélatine de type A.

8. Gélatine selon les revendications précédentes, dans laquelle la gélatine fonctionnalisée est en outre modifiée par un groupe ou un groupement (méth)acrylate, un groupe ou groupement acétyle, un groupe ou groupement phénol, un groupe ou groupement thiol, un groupe ou groupement norbornène, un groupe ou groupement tétrazine, un groupe ou groupement azoture, un groupe ou groupement furane, un groupe ou groupement allyle, un groupe ou groupement maléimide ou toute combinaison de ceux-ci.

9. Procédé de fourniture d'une gélatine fonctionnalisée selon l'une quelconque des revendications 1 à 8, le procédé comprend les étapes de
a. modification de gélatine par mise en réaction de gélatine avec un réactif contenant un ester de N-hydroxysuccinimide du groupement acide carboxylique R-(CH₂)ₙ-COOH où n est un entier de 1 à 10 (R-(CH₂)ₙ-COO-NHS) dans un milieu réactionnel ;
b. abaissement du pH du milieu réactionnel jusqu'à une valeur comprise entre 2,0 et 3,5 ;
c. ajout de 0,01-1,5 % p/p d'un tensioactif formant des micelles dans le milieu réactionnel acide ;
d. mise en contact du milieu contenant des micelles avec un adsorbant, de préférence du charbon actif ;
e. séparation de l'adsorbant du milieu ;
f. récupération du milieu comprenant la gélatine fonctionnalisée,
R étant choisi dans le groupe constitué par 4-phénol, norbornényle et SH.

10. Procédé de préparation d'une gélatine fonctionnalisée selon l'une quelconque des revendications 1 à 8, comprenant les étapes de :
a) modification de gélatine par mise en réaction de gélatine avec un réactif contenant un ester de N-hydroxysuccinimide du groupement acide carboxylique R-(CH₂)ₙ-COOH où n est un entier de 1 à 10 (R-(CH₂)ₙ-COO-NHS) dans un milieu réactionnel ;
b1). abaissement du pH du milieu réactionnel jusqu'à une valeur comprise entre 4,0 et 9,0, de préférence entre 4,0 et 6,0, de préférence encore entre 4,0 et 6,0, de préférence encore entre 4,5 et 5,5 ;
c) ajout de 0,01 -1,5 % p/p d'un tensioactif formant des micelles au milieu réactionnel ;
b2). abaissement du pH du milieu réactionnel jusqu'à une valeur comprise entre 2,0 et 3,5 ;
d). mise en contact du milieu de l'étape b2) avec un adsorbant solide ;
e). séparation de l'adsorbant solide de l'étape d) du milieu ; et
f). récupération du milieu comprenant la gélatine fonctionnalisée,
R étant choisi dans le groupe constitué par 4-phénol, norbornényle et SH.

11. Procédé selon la revendication 9 ou 10, dans lequel n est un entier de 1 à 5, de préférence 2 ou 3.

12. Procédé selon les revendications 9 à 11, dans lequel le réactif contenant un ester N-hydroxysuccinimide du groupement acide carboxylique R-(CH₂)ₙ-COOH dans lequel n est un entier de 1 à 10, est R-(CH₂)ₙ-COO-NHS.

13. Procédé selon les revendications 9 à 12, dans lequel le groupement acide carboxylique est un ou plusieurs parmi acide 3-(4-hydroxyphényl)-propionique, acide 3-(SH)-propionique et acide 2-(5-norbornényl)-acétique, de préférence acide 3-(4-hydroxyphényl)-propionique.

14. Procédé selon les revendications 9 à 13, dans lequel les groupes amine primaire de la gélatine sont modifiés par le groupement acide carboxylique.

15. Procédé selon les revendications 9 à 14, dans lequel un groupe amine primaire de la gélatine fonctionnalisée est couplé au groupement acide carboxylique via une liaison amide.

16. Procédé selon les revendications 9 à 15, dans lequel le procédé ne comprend pas une étape de dialyse.

17. Procédé selon l'une quelconque des revendications 9 à 16, dans lequel ledit tensioactif formant des micelles comprend un tensioactif non ionique, de préférence dans lequel le tensioactif est Triton X-100 ou Triton X-102, ou des mélanges de ceux-ci.

18. Procédé selon les revendications 9 à 17, dans lequel la gélatine fonctionnalisée a une teneur en lipopolysaccharide inférieure à 100 UE/g, de préférence inférieure à 50 UE/g, plus préférablement inférieure à 20 UE/g, encore plus préférablement inférieure à 10 UE/g, encore plus préférablement inférieure à 5 UE/g, encore plus préférablement inférieure à 2 UE/g, le plus préférablement inférieure à 1 UE/g.

19. Procédé selon les revendications 9 à 18, dans lequel la gélatine fonctionnalisée a un degré de modification par le groupement acide carboxylique compris entre 5 % et 100 %, de préférence entre 25 % et 100 %, plus préférablement entre 50 % et 100 %, encore plus préférablement entre 75 et 100 %.

20. Procédé selon les revendications 9 à 19, dans lequel la gélatine fonctionnalisée contient une quantité d'acide carboxylique libre R-(CH₂)ₙ-COOH inférieure à 100 ppm, de préférence inférieure à 50 ppm, plus préférablement inférieure à 20 ppm, encore plus préférablement inférieure à 10 ppm, encore plus préférablement inférieure à 5 ppm, encore plus préférablement inférieure à 2 ppm, de manière préférée entre toutes inférieure à 1 ppm,
dans lequel la quantité d'acide carboxylique libre R-(CH₂)ₙ-COOH est déterminée par chromatographie liquide haute performance sur un filtrat obtenu par dissolution de gélatine dans un tampon phosphate 50 mM, pH 9,5, et ultrafiltration de l'échantillon dissous.

21. Procédé selon les revendications 9 à 20, dans lequel la gélatine fonctionnalisée est en outre modifiée par un groupe ou groupement (méth)acrylate, un groupe ou groupement acétyle, un groupe ou groupement phénol, un groupe ou groupement thiol, un groupe ou groupement norbornène, un groupe ou groupement tétrazine, un groupe ou groupement azoture, un groupe ou groupement furane, un groupe ou groupement allyle, un groupe ou groupement maléimide ou toute combinaison de ceux-ci.

22. Procédé selon l'une quelconque des revendications 9 à 21, comprenant une étape de séchage du milieu comprenant la gélatine fonctionnalisée de l'étape f).

23. Hydrogel comprenant une gélatine fonctionnalisée telle que définie dans l'une quelconque des revendications 1 à 8 et un agent de réticulation.

24. Film comprenant un hydrogel selon la revendication 23 ou une gélatine fonctionnalisée selon les revendications 1 à 8.
